# EUROPEAN PATENT APPLICATION

(11) **EP 4 660 575 A1**
(43) Date of publication of application: **10.12.2025**
(21) Application number: 24179969.1
(22) Date of filing: 04.06.2024
(51) Int. Cl.: G01B 11/25, A61B 1/00, A61B 1/05, A61B 1/06

(54) **MEASURING HEAD, IN PARTICULAR FOR AN ENDOSCOPE**

(60) Divisional application: 25173556.9
(71) Applicant: ESPACE2001 S.A., 6496 Echternach (LU)
(72) Inventor: GAIL, Jonathan, 35685 Dillenburg (DE); BOLL, Alexander, 35606 Solms (DE); BAHR, Sönke Uwe, 64297 Darmstadt (DE); KRUSE, Felix, 22047 Hamburg (DE); LEDER, Günther, 68775 Ketsch (DE); NEDDERMEYER, Werner, 6496 Echternach (LU)
(74) Representative: Fuchs Patentanwälte Partnerschaft mbB

(57) **Abstract**

A measuring head (100; 330; 410; 600), in particular for an endoscope, comprises at least one laser device (110) configured to output a laser beam (L_O, L_D), and at least one mirror device (120) configured to receive the laser beam (L_O) output by the laser device (110) and to deflect the received laser beam (L O, L_D) towards a measuring field (M) of the measuring head (100). The at least one mirror device (120) comprises at least one variable mirror arrangement (122, 124) configured to deflect the received laser beam (L_O, L_D) towards the measuring field (M) in accordance with one or more dynamic projection patterns (DP1; DP2) for projecting the one or more dynamic projection patterns (DP1; DP2) towards the measuring field and is configured to provide at least one position signal (SP) indicative of a variable position of the at least one variable mirror arrangement (122, 124). The measuring head (1) further comprises at least one image sensor device (130; 130, 650) configured to receive reflected light (L_R) of the laser beam (L_D), the reflected light (L_R) reflected towards the measuring head (100; 330; 410; 600) by one or more objects (O) in the measuring field (M). The at least one image sensor device (130; 130, 650) is configured to provide at least one image sensor signal (SL1; SL1, SL3) indicative of a position of the received reflected light (L_R) of the laser beam (L_D) in an image plane of the image sensor device (130; 130, 650). The at least one position signal (SP) and the at least one image sensor signal (SL1; SL1, SL3) are configured to be indicative of a depth profile of the one or more objects (O) in the measuring field (M) in accordance with a triangulation based at least partially on the one or more dynamic projection patterns (DP1; DP2).

## Description

The disclosure relates to a measuring head, in particular for an endoscope. The disclosure further relates to an endoscope, a measuring system, and a method of operating a measuring head.

### Background of the Disclosure

Endoscopes, such as borescopes, are normally used for optical inspection of objects and/or regions with reduced mechanical and optical accessibility. Typical examples include human or animal body parts, hollow machine compartments, such as engine interiors, aircraft structures, etc., geological cavities, and the like. Endoscopes facilitate a mechanical and optical access to an interior of such structures, by allowing an operator to control the endoscope from outside the structure, and by having a relatively small extension in a cross-sectional plane perpendicular to an insertion direction of the endoscope, combined with light, in particular image, capturing facilities which are suitable for various practical applications.

Furthermore, optical measuring is in widespread use. Typical applications of optical measuring include quality assessment, for example, in the automated production of goods, etc., motion detection, for example, in connection with monitoring appliances, and/or technical inspection, for example, for determining deformation, abrasion or wear on mechanically exposed machine parts.

There is a continuous need for improved optical measuring and improved miniaturization of measuring devices.

### Summary of the Disclosure

Accordingly, there is provided a measuring head according to claim 1, an endoscope according to claim 12, a system according to claim 13, and a method according to claim 14.

According to an aspect, a measuring head, in particular for an endoscope, is provided. The measuring head comprises at least one laser device configured to output a laser beam, and at least one mirror device configured to receive the laser beam output by the laser device and to deflect the received laser beam towards a measuring field of the measuring head. The at least one mirror device comprises at least one variable mirror arrangement is configured to deflect the received laser beam towards the measuring field in accordance with one or more dynamic projection patterns for projecting the one or more dynamic projection patterns towards the measuring field and is configured to provide at least one position signal indicative of a variable position of the at least one variable mirror arrangement. The measuring head further comprises at least one image sensor device configured to receive reflected light of the laser beam, the reflected light reflected towards the measuring head by one or more objects in the measuring field. The at least one image sensor device is configured to provide at least one image sensor signal indicative of a position of the received reflected light of the laser beam in an image plane of the image sensor device. The at least one position signal and the at least one image sensor signal are configured to be indicative of a depth profile of the one or more objects in the measuring field in accordance with a triangulation based at least partially on the one or more dynamic projection patterns.

The at least one position signal and the at least one image sensor signal may be configured to be indicative of the depth profile in accordance with the triangulation, at least partially, by means of a spatial offset, with respect to the measuring field, between a deflection point of the laser beam on the at least one variable mirror arrangement and the position of the received reflected light of the laser beam in the image plane of the image sensor device.

By providing a laser beam, in combination with enabled triangulation, the measuring head facilitates measuring a depth profile of an object, including in poorly illuminated or unilluminated environments, in a contact free manner. Furthermore, by providing a laser beam in combination with enabled triangulation, the measuring head facilitates measuring a depth profile of objects having homogeneous surfaces, which are normally difficult, if not impossible, to be measured with respect to their depth profile by means of photographic imaging, especially stereographic imaging, due to the absence of suitable landmarks in the acquired image data of such objects. Furthermore, by providing a laser beam in combination with enabled triangulation, the measuring head dispenses with a requirement to illuminate large portions of the measuring field, as typically required for photographic imaging, thereby reducing an amount of electric energy and, accordingly, an amount of heat dissipation which is imported by the measuring head.

Furthermore, by providing a variable mirror arrangement for deflecting the laser beam towards the measuring field in accordance with one or more dynamic projection patterns, the measuring head facilitates determining a depth profile over an extended, especially areal, measuring field, without a necessity to re-orient the measuring head accordingly, by varying a deflection angle at which the laser beam is deflected by means of the at least one variable mirror arrangement.

Furthermore, by enabling the projection of a dynamic projection pattern, wherein triangulation based at least partially on the dynamic projection pattern is enabled, the measuring head facilitates adapting, in particular scaling and/or locally restricting, the available image sensor data.

This is achievable, as the measuring head facilitates applying triangulation to one or more characteristics of the dynamic projection pattern as captured by the image sensor device, thereby dispensing with a requirement to perform triangulation individually on each of multiple data sets of a particular position of the at least one variable mirror arrangement and an associated position of the received reflected light of the laser beam in the image plane of the image sensor device, respectively. Furthermore, by enabling the projection of a dynamic projection pattern, wherein triangulation based at least partially on the dynamic projection pattern is enabled, the measuring head facilitates adapting the available image sensor data to a given measurement requirement, by enabling that one or more characteristics of the dynamic projection pattern have been selected and/or controlled corresponding to the given measurement requirement by a user of the measuring head. Furthermore, by enabling the projection of a dynamic projection pattern, wherein triangulation based at least partially on the dynamic projection pattern is enabled, and by providing a position signal indicative of a variable position of the variable mirror arrangement, the measuring head facilitates that a precise active control of the position of the variable mirror arrangement can be dispensed with, thereby enabling an at least partially uncontrolled variation of the position of the variable mirror arrangement. This facilitates a simplified actuation of the variable mirror arrangement, which dispenses with a need for precisely controllable actuators. Moreover, by dispensing with a need for precisely controllable actuators of the variable mirror arrangement, improved miniaturization of the measurement head is also facilitated.

In the context of this disclosure, the term "dynamic projection pattern" denotes a geometric pattern which is non-statically projected, and which involves for its projection a movement of the laser beam by varying, during the projection, a position of the variable mirror arrangement. Additionally, "dynamic projection pattern" may denote a geometric pattern having one or more geometric characteristics, in particular one or more geometric boundaries of at least one area of the geometric pattern, whose projection involves shielding of the laser beam depending on the varying position of the variable mirror arrangement or whose projection involves dynamic activation and/or deactivation of the laser beam depending on the varying position of the variable mirror arrangement. In such case, the varying position of the variable mirror arrangement may vary at least partially in an uncontrolled, in particular a quasi-random, manner, and the laser beam may be shielded or may be dynamically activated and/or deactivated, respectively, depending on the at least partially uncontrolled position of the variable mirror arrangement, for projecting the geometric pattern. Additionally, or alternatively, "dynamic projection pattern" may denote a geometric pattern having one or more geometric characteristics, in particular one or more geometric boundaries of at least one area of the geometric pattern, which are variable during an operation of the measuring head.

The at least one variable mirror arrangement may comprise an oscillating mirror arrangement configured to produce an oscillating deflection plane for the laser beam. The dynamic projection pattern may be an areal dynamic projection pattern and the oscillating deflection plane may be configured to oscillate about at least two non-parallel axes for deflecting the laser beam towards the measuring field in accordance with the areal dynamic projection pattern. An oscillation of the deflection plane about a first one of the at least two non-parallel axes may have a first periodicity, and an oscillation of the deflection plane about a second one of the at least two non-parallel axes may have a second periodicity different from the first periodicity. Additionally, or alternatively, the dynamic projection pattern may comprise at least one Lissajous curve.

The variable mirror arrangement may comprise one or actuators configured to drive one or more oscillations of the oscillating mirror arrangement.

The variable position of the at least one variable mirror arrangement may be variable to vary a propagation direction of the deflected laser beam over a range of 30 degrees or more, particularly 45 degrees or more, more particularly 60 degrees or more.

The variable mirror arrangement may be a micro-electro-mechanical system, MEMS. By implementing the variable mirror arrangement as a micro-electro-mechanical system, MEMS, improved miniaturization of the measurement head is further facilitated.

The oscillating mirror arrangement may be configured to oscillate about each of the at least two non-parallel axes at a frequency in a range between 10 hertz and 20 kilohertz, in particular between 1 and 10 kilohertz, more particularly between 2 and 6 kilohertz.

Additionally, or alternatively, the oscillating mirror arrangement may provide an oscillating deflection plane having a largest diameter in a range between 1 and 8 millimeter, in particular between 2 and 5 millimeter, more particularly between 2.5 and 4 millimeter.

The variable mirror arrangement may be configured to be operated with respect to each of the at least two non-parallel axes by a sinusoidal voltage in a range between +/-1 and +/- 100 volts, in particular between +/-1 volts and +/- 40 volts, more particularly between +/- 2 and +/- 20 volts.

The measuring head may have a largest diameter less than 12 millimeters, particularly less than 10 millimeters, more particularly less than 8 millimeters, in every cross-sectional plane extending perpendicularly to a longitudinal axis of the measuring head. The longitudinal axis of the measuring head may be parallel to an insertion direction of the measuring head and/or of an endoscope in which the measuring head is to be mounted, the insertion direction corresponding to a direction in which the measuring head is to be inserted into a volume for measuring. In this way, a conformity of the measuring head with spatial restrictions posed by technical conditions and/or by legal regulations in various technical fields and/or jurisdictions, such as concerning a size of inspection ports on aircraft engines, is improved.

Additionally, or alternatively, a center of the measuring field as seen from the measuring head may be in a direction non-parallel to, in particular essentially perpendicular to, the longitudinal axis of the measuring head.

The at least one laser device may comprise a switchable laser device. Additionally, projecting the one or more dynamic projection patterns towards the measuring field by means of the measuring head may further comprise switching the switchable laser device based at least partially on the variable position of the at least one variable mirror arrangement.

By switching the switchable laser device based at least partially on the variable position of the at least one variable mirror arrangement, one or more geometric boundaries of the dynamic projection pattern may be dynamically projected. Additionally, or alternatively, one or more geometric boundaries of the dynamic projection pattern may be dynamically produced by means of an optical shielding arrangement of the measuring head.

The measuring head may further comprise at least one support structure on which the at least one laser device, the at least one mirror device and the at least one image sensor device are arranged.

The measuring head may be configured to output the at least one position signal and the at least one image sensor signal towards a data processor of a measuring system to which the measuring head is operatively couplable.

The at least one image sensor device may be further configured for photographic imaging of the one or more objects in the measuring field. The at least one image sensor device may comprise a color image sensor, in particular a red-green-blue, RGB, color image sensor.

By additionally enabling photographic imaging of objects in the measuring field, photographic detection of local structures in the measuring field which may affect an applicability of triangulation and/or a significance of triangulation results is facilitated.

Additionally, the measuring head may further comprise an illumination device, in particular a white light source, configured to illuminate the one or more objects in the measuring field. The illumination device may be configured to illuminate the one or more objects in the measuring field when the measuring head is operated in a photographic imaging mode. In this way, improved photographic imaging of the one or more objects in the measuring field is enabled.

Additionally, or alternatively, the at least one image sensor device may comprise a first image sensor device and at least a second image sensor device arranged spatially offset from the first image sensor device with respect to the measuring field. Image sensor signals of the first image sensor device and the second image sensor device may be further configured to be indicative of the depth profile of the one or more objects in the measuring field in accordance with a stereography based at least partially on the spatial offset between the first image sensor device and the second image sensor device.

Additionally, or alternatively, the measuring head may further comprise a photo diode, in particular an avalanche photo diode, more particularly a single-pixel avalanche photo diode. The photo diode may be configured to receive reflected light of the laser beam, the reflected light reflected towards the measuring head by the one or more objects in the measuring field. The photo diode may be configured to provide at least one photo diode signal indicative of at least a time at which the reflected light of the laser beam has been received by means of the photo diode, wherein the at least one position signal and the at least one photo diode signal are configured to be indicative of the depth profile of the one or more objects in the measuring field in accordance with a time of flight.

The time of flight may be determinable based at least partially on a phase shift of the received reflected light of the laser beam. In such case, the laser beam may be a continuous, sine-modulated laser beam.

Alternatively, the time of flight may be determinable based at least partially on a time difference between an output of the laser beam by the laser device and a detection of the reflected light of the laser beam by the photodiode. In such case, the laser beam may be a pulsed laser beam.

By enabling that the depth profile of the one or more objects in the measuring field is additionally determinable by means of stereography and/or based on a time of flight, redundancy of available depth-related information and of applicable methods of determining the depth profile is enabled. This enables improved depth measurements in situations in which triangulation is disadvantageous and/or not applicable. Moreover, it enables improved measurements of a depth profile by permitting improved error reduction by enabling that the redundant depth-related information and/or any results obtained by the redundant applicable methods become correlated to each other in a data processing procedure. Correlating to each other the redundant depth-related information and/or any results obtained by the redundant applicable methods may comprise averaging any of the redundant depth-related information and/or any results obtained by the redundant applicable methods. Additionally, or alternatively, correlating to each other the redundant depth-related information and/or any results obtained by the redundant applicable methods of determining a depth profile may comprise comparing with each other the redundant depth-related information and/or any results obtained by the redundant applicable methods to determine a plausibility of the depth-related information and/or of the obtained results.

The at least one laser device may comprise at least one laser diode. Alternatively, the at least one laser device may be configured to receive the laser beam from a laser source external to the measuring head and to output the received laser beam towards the at least one mirror device. Additionally, or alternatively, the at least one laser device may comprise at least one collimation lens.

The at least one laser device may be configured to output a pulsed laser beam. Additionally, or alternatively, the at least one laser device may be configured to output a continuous laser beam.

The laser beam may have a power in the range between 5 and 50 milliwatts, particularly between 10 and 20 milliwatts. Additionally, or alternatively, the laser beam may have a wavelength in the range between 450 and 600 nanometers, particularly between 500 and 550 nanometers. Additionally, or alternatively, the laser beam may have a diameter in the range between 50 and 150 micrometers at a measuring distance of 500 millimeters.

The measuring head may further comprise an inertial sensor device configured to detect a linear and/or a rotational acceleration of the measuring head and to provide at least one inertial sensor signal indicative of a detected linear and/or rotational acceleration of the measuring head. By providing the inertial sensor signal, compensating a motion of the measuring head during a measurement by means of data processing is facilitated.

According to another aspect, an endoscope is provided. The endoscope comprises a measuring head as provided herein. The endoscope may be a borescope.

According to another aspect, a measuring system is provided. The measuring system comprises a measuring head as provided herein and/or an endoscope as provided herein comprising such measuring head. The measuring system further comprises a data processor operatively connected to the measuring head. The data processor is configured to receive, from the measuring head, at least one position signal indicative of a variable position of the at least one variable mirror arrangement and at least one image sensor signal indicative of a position of received reflected light of the laser beam in an image plane of the image sensor device, and to process the received at least one position signal and the received at least one image sensor signal in accordance with a triangulation based at least partially on the one or more dynamic projection patterns to determine a depth profile of one or more objects in the measuring field.

According to another aspect, a method of operating a measuring head, in particular for an endoscope, is provided. The method comprises providing a measuring head as provided herein; positioning the measuring head such that one or more objects to be measured are in the measuring field of the measuring head; receiving, from the measuring head, at least one position signal indicative of a variable position of the at least one variable mirror arrangement and at least one image sensor signal indicative of a position of received reflected light of the laser beam in an image plane of the image sensor device; and, processing, by means of a data processor, the received at least one position signal and the received at least one image sensor signal in accordance with a triangulation based at least partially on the one or more dynamic projection patterns to determine a depth profile of the one or more objects.

The at least one image sensor signal may be further indicative of one or more photographic characteristics, in particular one or more colors, of the one or more objects in the measuring field. Additionally, processing the received at least one position signal and the received at least one image sensor signal may further comprise associating the one or more photographic characteristics with the depth profile of the one or more objects to generate at least one data set comprising a three-dimensional photographic representation of at least a portion of the one or more objects.

Processing the received at least one position signal and the received at least one image sensor signal may be performed in accordance with a calibration rule for the measuring head.

### Brief Description of the Drawings

Further details and advantages of the disclosure will be apparent from the drawings and the detailed description. There is shown in:
- Fig. 1: a measuring head according to an example;
- Figs. 2A, 2B: dynamic projection patterns according to different examples;
- Fig. 3: an endoscope according to an example;
- Fig. 4: a measuring system according to an example;
- Fig. 5: a method of operating a measuring head according to an example; and
- Fig. 6: a measuring head according to another example.

### Detailed Description

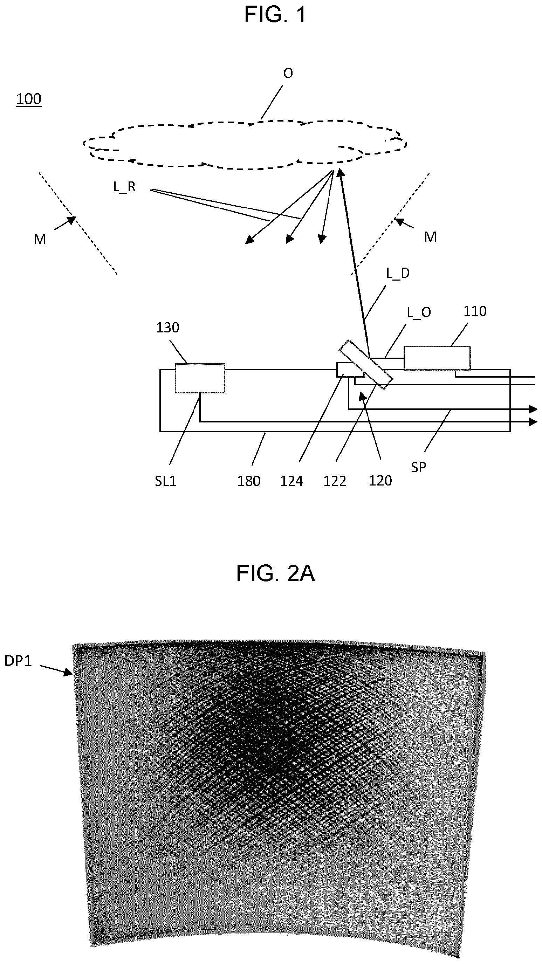

Fig. 1 shows schematically and exemplarily a measuring head 100, for example, for use in an endoscope, such as a borescope. The measuring head 100 comprises a laser device 110 which outputs a laser beam L_O. The measuring head 100 further comprises a mirror device 120, comprising a light deflecting element 122 and an actuator 124 configured to change a position of the light deflecting element 122. The light deflecting element 122 and the actuator 124 constitute a variable mirror arrangement of the mirror device 120. The measuring head 100 further comprises an image sensor device 130. The laser device 110, the variable mirror arrangement 122, 124 and the image sensor device 130 are arranged on a support structure 180 of the measuring head 100.

The laser device 110 and the variable mirror arrangement 122, 124 are arranged such that the mirror device 120 receives the laser beam L_O output by the laser device 110 at the light deflecting element 122. The light deflecting element 122 is configured to deflect the received laser beam L_O, L_D towards a measuring field M of the measuring head 100.

During operation of the measuring head 100, when an object O is in the measuring field M, the deflected laser beam L_D impinges on the object O and is reflected at the object O, for example, at a surface of the object O facing towards the measuring head 100. Furthermore, in typical applications, as shown in Fig. 1, reflection of the deflected laser beam L_D at the object O coincides with scattering of the laser light, whereby portions of the scattered reflected light L_R are reflected towards the measuring head 100.

The image sensor device 130 receives and detects some of the reflected light L_R and detects a position in an image plane, for example, on a planar array of sensor pixels, of the image sensor device 130 at which the received reflected light L_R is detected. Furthermore, the image sensor device 130 generates and outputs an image sensor signal SL1 in which information on the position of the received reflected light L_R in the image plane of the image sensor device 130 is encoded.

The mirror device 120 is configured to automatically vary, by varying the position of the light deflecting element 122, a direction in which the laser beam L_O, L_D is deflected during operation of the measuring head 100. Furthermore, the mirror device 120 is configured to output, for example, continuously or at intervals, a position signal SP which is indicative of the variable position of the mirror arrangement 120, 122. For example, the position signal SP is indicative an orientation of a surface normal of the light deflecting element 122, or inclination angles of the light deflecting element 122 about each of at least two non-parallel axes with respect to a reference inclination of the light deflecting element 122 about each of these axes, etc.

As can be understood from Fig. 1, when a position of the light deflecting element 122 is varied by means of the actuator 124, a deflection angle of the deflected laser beam L_D changes correspondingly, and the deflected laser beam L_D will be reflected at a different point of the object O. This typically causes that the reflected light L_R is detected at a different position in the image plane of the image sensor device 130. As can be further understood from Fig. 1, for any given distance of reflection points in the measuring field M, such as on object O, from the measuring head 100, each position of the light deflecting element 122 typically corresponds to a particular position in the image plane of the image sensor device 130 at which the reflected light L_R is detected. As can be further understood from Fig. 1, when a distance between the object O and the measuring head 100 varies, a point at which the deflected laser beam L_D is reflected at the object O typically also changes for any given direction of the deflected laser beam L_D, and, due to a spatial offset between the mirror device 120 and the image sensor device 130, a position at which the reflected light L_R is detected in the image plane of the image sensor device 130 also changes.

Consequently, when the relationship between any of multiple directions of the deflected laser beam L_D, a position at which the reflected light L_R is detected in the image plane of the image sensor device 130, and a distance of the reflection point in the measuring field M from the measuring head 100 is known, the image sensor signal SL1 and the position signal SP are indicative of the distance of the corresponding reflection point at the object O, according to a depth profile of the object O, by way of triangulation. In some examples, the relationship is determined based on known dimensions of the measuring head 100, by means of an initial calibration of the measuring head 100, etc. Furthermore, in some examples, the relationship is stored in a storage device of the measuring head (not shown), in a storage device of an external measuring system to which the measuring head 100 is couplable, etc.

The position of the light deflecting element 122 is variable about two non-parallel axes of the light deflecting element 122. Additionally, the image sensor device 130 is configured for acquisition of two-dimensional image information. Consequently, the measuring field M covers a solid angle, and an areal depth profile of the object O is measurable, by means of the measuring head 100.

As shown schematically by the horizontal arrows in Fig. 1, the measuring head 100 is configured to output the image sensor signal SL1 and the position signal SP, for example, towards a data processor which has been programmed to process the signals SL1, SP to derive from these signals a depth profile of the object O, in accordance with the above-described triangulation. To this end, the measuring head 100 is operationally couplable to a data processor, as described in more detail below.

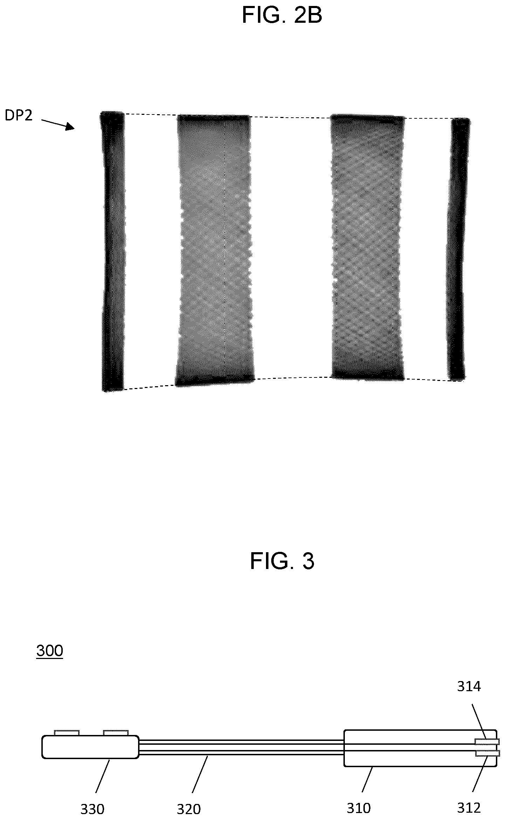

As schematically shown in Fig. 1, the mirror device 120 is configured to receive a control or activation signal, for example, from a control device of a measuring system, as described in more detail below. Based on the received control or activation signal, the mirror device 120 is configured to deflect the laser beam L_O towards the measuring field M in accordance with a dynamic projection pattern, as described in more detail in connection with Figs 2A and 2B.

In some examples, the variable mirror arrangement 122, 124 is an oscillating mirror arrangement. That is, the actuator 124 is configured to drive the light deflecting element 122 such that the position of the light deflecting element 122 varies by oscillation. In addition, the mirror device 120 is configured to drive the light deflecting element 122 such that the light deflecting element 122 oscillates about two non-parallel axes. In some of these examples, an oscillation about a first one of the two non-parallel axes has a different periodicity than an oscillation about the other, i.e., second, axis. As described in more detail in connection with Figs 2A and 2B, by suitable selection of the oscillation frequencies, this facilitates a quasi-areal coverage of the measuring field M, or parts thereof, by deflecting the laser beam L_D according to one or more Lissajous curves, without a requirement of an active and precise position control of the light deflecting element 122.

In some examples, the variable mirror arrangement 122, 124 is a micro-electromechanical system, MEMS. Implementing the variable mirror arrangement 122, 124 as a MEMS facilitates a miniaturized implementation of a measuring head 100 which is capable of covering a two-dimensional measuring field M. In particular, by implementing the variable mirror arrangement 122, 124 as a MEMS, it is possible to minimize a diameter of the measuring head 100 perpendicular to an insertion direction of the measuring head 100.

In some examples, the measuring head has a largest diameter of less than 12 mm, particularly less than 10 mm, more particularly less than 8 mm, in every cross-sectional plane extending perpendicularly to a longitudinal axis of the measuring head 100. In some of these examples, the longitudinal axis of the measuring head 100 extends parallel to an intended insertion direction for inserting the measuring head 100 into a measuring zone.

In some examples, the laser device 110 is switchable between an activated state, in which the laser beam L_O is output by the laser device 110, and a deactivated state, in which a laser beam L_O is not output by the laser device 110. As described in more detail below in connection with Figs. 2A and 2B, a switchable laser device 110 facilitates switching the laser device 110 in accordance with the variable position of the mirror arrangement 122, 124 for projecting a dynamic projection pattern towards the measuring field M.

In some examples, the image sensor device 130 is additionally configured for photographic imaging, for example, color image acquisition. In some of these examples, the image sensor device 130 includes a color image sensor, such as an RGB image sensor. In some examples, when suitable illumination conditions exist in the measuring field M, photographic imaging by means of the image sensor device 130 is usable for supplementing the depth-related information contained in the image sensor signal SL1 with color information on the measured object O. Furthermore, in some examples, photographic imaging by means of the image sensor device 130 is used for determining whether the object O exhibits characteristics which potentially affect, such as deteriorate, an applicability or significance of triangulation as described above.

In some examples, the laser device 110 includes a laser diode. Additionally, or alternatively, in some examples, the measuring head 100, such as the laser device 110, comprises a collimation lens (not shown) for the laser beam. In some examples, the laser device 110 outputs a pulsed laser beam L_0 or a continuous laser beam L_D.

Figs 2A and 2B show examples of dynamic projection patterns DP1, DP2 in accordance with the present disclosure. The dynamic projection patterns DP1, DP2 have been projected onto example object surfaces, and detected, by means of a measuring head, such as measuring head 100. The mirror device for projecting the dynamic projection patterns DP1, DP2 was implemented as a MEMS with an oscillating deflection plane having different oscillation frequencies about two orthogonal axes. In both Figs. 2A and 2B, the laser beam has been deflected in accordance with a single Lissajous curve. The different oscillation frequencies of the mirror device were chosen such that the resulting Lissajous curve provides for a quasi-areal coverage of an entire area of the dynamic projection pattern. A brightness contrast of the detected pattern area(s) has been inverted in Figs. 2A and 2B, for convenience.

Fig. 2A shows dynamic projection pattern DP1, which resembles a coherent quadrilateral area. In the shown example, geometric characteristics, in particular geometric borders, of the dynamic projection pattern DP1 are at least partially produced by, and correspond at least partially to, a maximal deflection of the laser beam towards the corresponding direction(s) by means of the variable mirror arrangement. In other examples, geometric borders of the dynamic projection pattern DP1 are at least partially produced by shielding of the laser beam and/or by controlled switching of the laser device, based on the position signal output by the mirror device, when the laser beam enters or leaves the quadrilateral area.

In the example shown in Fig. 2A, the sides of the quadrilateral, as seen from the image sensor, are slightly curved. The curvature of the sides of the quadrilateral corresponds, for example, to a deviation of the object surface from a reference plane in which the dynamic projection pattern DP1 is configured to appear as a regular, i.e., a rectangular and straight, quadrilateral. A deviation as seen in Fig. 2A results in such case, for example, from a depth profile of the example object plane which deviates from a depth profile of the reference plane. Deviations as shown in Fig. 2A result, for example, when the reference plane for which the measuring head was calibrated is a flat plane, and the example object plane has a depth profile corresponding to a domed shape.

By comparing the detected shape of the dynamic projection pattern DP1 with a stored reference shape of the dynamic projection pattern DP1, a depth profile of the object surface can thus be determined, using triangulation, as described above. Furthermore, by using geometric characteristics of the dynamic projection pattern DP1, such as geometric borders of one or more pattern areas, an individual position of the laser beam at any moment does not need to be considered for determining the depth profile. This allows for a use of a partially uncontrolled, for example, quasi-random, variation of a position of the variable mirror arrangement, thus facilitating a simplified and/or miniaturized implementation and operation of the variable mirror arrangement, such as an oscillating MEMS. Additionally, in such way, the projection of the dynamic projection pattern DP1 allows for determining a depth profile more efficiently in many applications. Furthermore, geometric characteristics of the dynamic projection pattern DP1 can be selected, and/or varied, by selecting and/or varying the dynamic projection pattern, depending on a measurement requirement, such as a resolution requirement and/or a local restriction to a particular region of the object surface, without demanding precise active position control of the variable mirror arrangement.

Fig. 2B shows another example of a dynamic projection pattern DP2. Different from DP1, the dynamic projection pattern DP2 consists of multiple quadrilateral areas which have been covered in a quasi-areal manner by the deflected laser beam along a Lissajous curve. A movement of the MEMS is performed with the same settings as in Fig. 2A. Moreover, the separate quadrilateral areas are produced by controlled switching of the laser device when the laser beam enters or leaves any of the areas. The controlled switching of the laser device is performed based on the position signal output by the mirror device, in accordance with an initially performed, and stored, calibration of the measuring head. By controlled switching of the laser device, the dynamic projection pattern DP2 can also be varied in some examples, for examples, be rotated, re-scaled, etc., during a measurement. This is advantageous, for example, when different topographic characteristics of the object surface are to be measured and/or when different projection patterns are differently suitable for measuring different characteristics of the object.

In the example of Fig. 2B, the dynamic projection pattern DP2 contains multiple rectangular and straight quadrilateral areas when projected onto a reference object surface, as seen from the image sensor device. Furthermore, similarly to the example in Fig. 2A, in the example of Fig. 2B any deviations of the quadrilateral areas of the pattern as detected by the image sensor device from rectangular and straight shapes is indicative, by way of triangulation, of a depth profile of the example object surface relative to the reference object surface.

Fig. 3 shows schematically and exemplarily an endoscope 300, for example, a borescope. The endoscope 300 comprises at a proximal end a handling portion 310 which is configured to be held by a user of the endoscope 300 and which is configured for manipulating the endoscope 300 before, during, and after a measurement. Attached to the handling portion 310 is a shaft 320 of the endoscope 300. At a distal end of the shaft 320, a measuring head 330 of the endoscope 300 is arranged. The measuring head 330 is a measuring head according to one or more of the examples described herein.

At the handling portion 310, an input interface 312 and an output interface 314 of the endoscope 300 are schematically shown. Furthermore, as schematically shown, the input interface 212 and the output interface 314 are operatively coupled to the measuring head 330 via electrically and/or optically conductive lines to facilitate the transmission of data and/or operational supply, such as a supply laser light from an external laser source, between the interfaces 312, 314 and one or more functional components of the measuring head 330. The interfaces 312, 314 enable operatively coupling the endoscope 300 to a data processor and, in some examples, to a supply unit for operating the endoscope 300.

One input interface 312 and one output interface 314 of the endoscope 300 are shown in Fig. 3. However, in other examples, a larger number of input interfaces and/or output interfaces of the endoscope 300 are provided, in accordance with requirements.

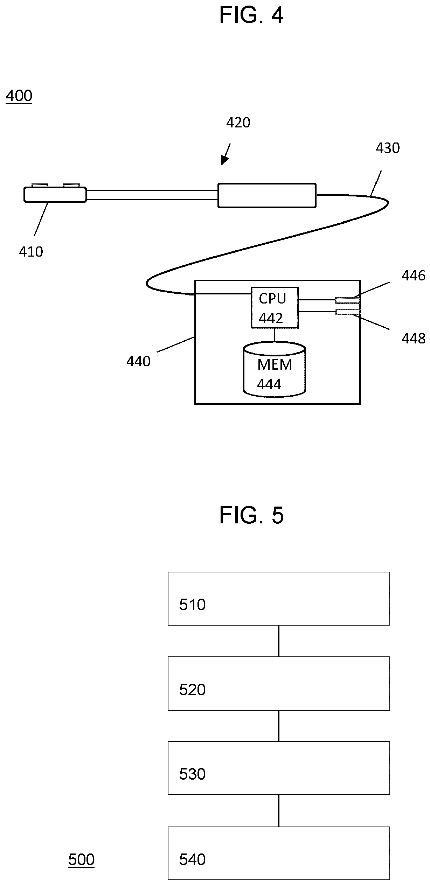

Fig. 4 shows schematically and exemplarily a measuring system 400. The measuring system 400 comprises a measuring head 410 according to any of the examples described herein. The measuring head 410 is operatively coupled to a data processor 442 by means of a cable 430. In the shown example, the measuring head 410 is part of an endoscope 420 of the measuring system 400. The endoscope 420 is in some examples an endoscope in accordance with any of the examples described in connection with the endoscope 300 shown in Fig. 3. Furthermore, in the shown example, the coupling of the measuring head 410 to the data processor 442 by means of the cable 430 includes a coupling of the endoscope 420 to the cable 430.

The data processor 442 is arranged in a processing device 440 of the measuring system 400, for example, in a stationary or mobile computer device. In addition, the data processor 442 is operatively coupled to a data storage device 444 and to each of an input interface 446 and an output interface 448 of the processing device 440.

The data processor 442 is configured to receive, via the cable 430, sensor information which has been obtained by means of the one or more sensor devices of the measuring head 410, and to output, via the cable 430, control signals for operating the measuring head 410. The data processor 442 is further configured to process the received sensor information for determining a depth profile of an object in a measuring field of the measuring head 410 in accordance with any of the examples described herein. The data processor 442 is in some examples configured to output the determined depth profile to the output interface 448 of the processing device 440, for example, for external storage, remote communication, or visual display of a graphical representation of the determined depth profile by means of a display device (not shown), and/or to store the determined depth profile in the data storage device 444 for later use.

In some examples, operational information for operating the measuring head 410 are additionally stored in the data storage device 444. Operational information of such type includes, in some examples, one or more dynamic projection pattern control data for operating the measuring head 410 in accordance with corresponding dynamic projection patterns, as described herein.

In some examples, the data storage device 444 further contains executable program code which is accessible, and executable, by means of the data processor 442 to configure the data processor 442 for executing one or more of the methods described herein of operating the measuring head 410.

In some examples, the input interface 446 is provided for providing the processing device 440 with updated data concerning one or more dynamic projection patterns, which in some examples will be stored in the data processing device 444 and/or will be executed in real time by means of the data processor 442 during an operation of the measuring head 410. In other examples, additional and/or other operational data is provided to the data processor 442 via the input interface 446.

Fig. 5 shows a flow diagram of a method 500 of operating a measuring head. The method 500 is executable using a measuring head in accordance with any of the examples described herein.

The method 500 comprises providing the measuring head, step 510, and positioning the measuring head such that one or more objects to be measured are in a measuring field of the measuring head, step 520.

The method 500 further comprises receiving at least one position signal and at least one image sensor signal from the measuring head, step 530, in accordance with any of the examples described herein. The position signal is indicative of a variable position of a variable mirror arrangement of the measuring head. The image sensor signal is indicative of a position of received reflected light of a laser beam in an image plane of the image sensor device of the measuring head.

The method 500 further comprises processing the received position signal and the received image sensor signal in accordance with a triangulation based at least partially on one or more dynamic projection patterns to determine a depth profile of the one or more objects, step 540. For example, steps 530, 540 are executable by means of a data processor, such as data processor 442 of the measuring system 400.

In some examples of the method 500, the at least one image sensor signal is further indicative of one or more photographic characteristics of the one or more objects in the measuring field. In some of these examples, processing the received at least one position signal and the received at least one image sensor signal comprises associating the one or more photographic characteristics with the depth profile of the one or more objects. In this way, in some examples, a data set comprising a three-dimensional photographic representation of at least a portion of the one or more objects is generated.

In some examples of the method 500, the received at least one position signal and the received at least one image sensor signal are processed by means of the data processor in accordance with a calibration rule for the individual measuring head.

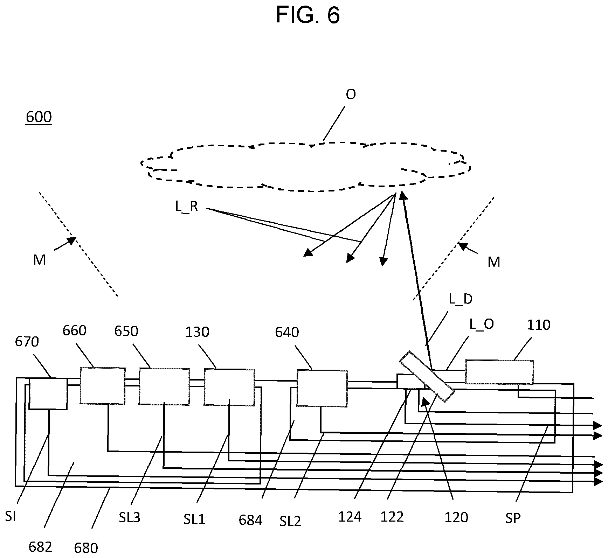

Figure 6 shows schematically and exemplarily a measuring head 600 according to another example. Components of the measuring head 600 which are structurally and functionally identical or similar to components of the measuring head 100 are denoted by the same reference numbers. Furthermore, the description of the measuring head 100 applies correspondingly to the measuring had 600 with respect to these components unless differently clear from the drawings and the following description.

Different from the measuring head 100, the measuring head 600 additionally comprises a photodiode 640, a second image sensor device 650, an illumination device 660, and an inertial sensor device 670, which are arranged on a correspondingly configured support structure 680 of the measuring head 600. The measuring head 600 further comprises printed circuit boards 682, 684, PCB, on which the mirror device 120, the image sensor devices 130, 650, the photodiode 640, the illumination device 660, and the inertial sensor device 670 are arranged. In the shown example, the mirror device 120 and the photodiode 640 are arranged on a first PCB 684, and the image sensor devices 130, 650, the illumination device 660, and the inertial sensor device 670 are arranged on a second PCB 682.

The photodiode 640 is configured to receive and detect reflected light L_R of the deflected laser beam L_D which is reflected at the object O towards the photodiode 640. In some examples, the photodiode 640 is implemented as an avalanche photodiode. The photodiode 640 is configured to output a photodiode signal SL2 which is indicative of light L_R which has been received and detected by the photodiode 640, for example, an intensity of the detected light L_R. Furthermore, the photodiode signal SL2 is indicative of a time at which the reflected light L_R of the laser beam L_D has been received and detected by the photodiode 640. In some examples, the photodiode signal SL2 includes a timestamp indicating the time at which the light L_R has been detected. In other examples, the photodiode signal SL2 is indicative of the time based on a, for example, a predetermined, temporal relationship between the moment when light L_R is detected by the photodiode 640 and the moment at which a corresponding photodiode signal SL2 is output.

The photodiode signal SL2 facilitates determining a depth profile of the object O in accordance with a time of flight. For example, in combination with a suitable control of the laser device 110, the photodiode signal SL2 enables determining a time of flight of light from the laser device 110, via the mirror device 120, reflection at object O, until detection by the photodiode 640. The time of flight varies according to a distance of the reflection point on object O, thus permitting determining a depth, in particular a depth profile, of the object O.

The second image sensor device 650 is arranged spatially offset from the image sensor device 130, which in this case constitutes a first image sensor device 130 of the measuring head 600. In some examples, the second image sensor device 650 is implemented structurally and functionally identical or similar to the first image sensor device 130. The second image sensor device 650 is likewise configured to receive reflected light L_R of the deflected laser beam L_D which has been reflected towards the measuring head 600 at the object O. Moreover, the second image sensor device 650 is likewise configured to output a second image sensor signal SL3 which is indicative of a position on an image plane of the second image sensor device 650 at which the reflected light L_R has been detected by the second image sensor device 650.

The second image sensor device 650 provides in some examples for the same functionalities to determine a depth profile of the object O using triangulation and, in some examples, a photographic imaging functionality, as described above for the first image sensor device 130.

In addition, or alternatively, in examples in which the first and the second image sensor devices 130, 650 both provide for a photographic imaging functionality, the first image sensor signal SL1 and the second image sensor signal SL3 are usable for stereographically determining a depth profile of the object O, based on a spatial offset of the image sensor devices 130, 650 from each other with respect to the measuring field M.

As described, the photodiode 640 and the second image sensor device 650 provide for additional means to determine a depth profile of the object O independently from a triangulation based on the first image sensor signal SL1 from the first image sensor device 130. As such, the measuring head 600 permits simultaneous and/or alternative performance of independent determinations of a depth profile. This enables, for example, that thus obtained independent results be compared, or otherwise related, to each other, for example, by means of a data processor of a measuring system, for data improvement, such as error detection, plausibility check, data averaging, etc. Furthermore, by additionally providing for time-of-flight-based and/or stereography-based determination of the depth profile, the measuring head 600 provides redundancy with respect to available measuring techniques. This enables flexible selection of the most suitable technique(s), for example, depending on optical characteristics and/or a topography of the object O, illumination conditions, etc.

The illumination device 660 is configured to illuminate the measuring field M. In some examples, the illumination device 660 is activated when the measuring head 600 is operated in a photographic imaging mode, that is, when one or both image sensor devices 130, 650 are operated to perform photographic imaging. In such examples, the illumination device 660 provides for improved image acquisition, for example, in poorly illuminated environments.

The inertial sensor device 670 is configured to detect translational and/or rotational acceleration of the measuring had 600 and to output a corresponding inertial sensor signal SI indicative of a detected acceleration. Based on the inertial sensor signal SI, movements of the measuring head 600 can be determined and compensated for, for example, by means of data processing. This allows for eliminating artifacts which are due to movements of the measuring head 600 during a measurement, for example, during a time span which is needed for projecting the dynamic projection pattern towards the measuring field M.

In the above examples, measuring heads have been described in the particular context of endoscopes. However, it will be understood that most or all of the described advantages of a measuring head according to the disclosure are also achievable in other contexts.

## Claims

1. Measuring head (100; 330; 410; 600), in particular for an endoscope, the measuring head (100; 330; 410; 600) comprising:
- at least one laser device (110) configured to output a laser beam (L_O, L_D);
- at least one mirror device (120) configured to receive the laser beam (L_O) output by the laser device (110) and to deflect the received laser beam (L_O, L_D) towards a measuring field (M) of the measuring head (100), wherein the at least one mirror device (120):
∘ comprises at least one variable mirror arrangement (122, 124) configured to deflect the received laser beam (L_O, L_D) towards the measuring field (M) in accordance with one or more dynamic projection patterns (DP1; DP2) for projecting the one or more dynamic projection patterns (DP1; DP2) towards the measuring field, and
∘ is configured to provide at least one position signal (SP) indicative of a variable position of the at least one variable mirror arrangement (122, 124), and
- at least one image sensor device (130; 130, 650) configured to receive reflected light (L_R) of the laser beam (L_D), the reflected light (L_R) reflected towards the measuring head (100; 330; 410; 600) by one or more objects (O) in the measuring field (M),
wherein the at least one image sensor device (130; 130, 650) is configured to provide at least one image sensor signal (SL1; SL1, SL3) indicative of a position of the received reflected light (L_R) of the laser beam (L_D) in an image plane of the image sensor device (130; 130, 650),
wherein the at least one position signal (SP) and the at least one image sensor signal (SL1; SL1, SL3) are configured to be indicative of a depth profile of the one or more objects (O) in the measuring field (M) in accordance with a triangulation based at least partially on the one or more dynamic projection patterns (DP1; DP2).

2. Measuring head according to claim 1, wherein the at least one variable mirror arrangement (122, 124) comprises an oscillating mirror arrangement configured to produce an oscillating deflection plane (122) for the laser beam (L).

3. Measuring head according to claim 2, wherein the dynamic projection pattern is an areal dynamic projection pattern and the oscillating deflection plane (122) is configured to oscillate about at least two non-parallel axes for deflecting the laser beam (L) towards the measuring field (M) in accordance with the areal dynamic projection pattern,
Wherein, as an option, an oscillation of the deflection plane (122) about a first one of the at least two non-parallel axes has a first periodicity, and an oscillation of the deflection plane (122) about a second one of the at least two non-parallel axes has a second periodicity different from the first periodicity.

4. Measuring head according to any one of the preceding claims, wherein:
- the variable mirror arrangement (122, 124) is a micro-electro-mechanical system, MEMS, and/or
- the measuring head has a largest diameter less than 12 millimeters, particularly less than 10 millimeters, more particularly less than 8 millimeters, in every cross-sectional plane extending perpendicularly to a longitudinal axis of the measuring head.

5. Measuring head according to any one of the preceding claims, wherein the at least one laser device (110) comprises a switchable laser device (110), and wherein projecting the one or more dynamic projection patterns (DP1; DP2) towards the measuring field (M) by means of the measuring head further comprises switching the switchable laser device (110) based at least partially on the variable position of the at least one variable mirror arrangement (122, 124).

6. Measuring head according to any one of the preceding claims, further comprising at least one support structure (180; 680) on which the at least one laser device (110), the at least one mirror device (120) and the at least one image sensor device (130; 130, 650) are arranged.

7. Measuring head according to any one of the preceding claims, wherein the measuring head (100; 330; 410; 600) is configured to output the at least one position signal (SP) and the at least one image sensor signal (SL1; SL1, SL3) towards a data processor (442) of a measuring system (440) to which the measuring head (100; 330; 410; 600) is operatively couplable.

8. Measuring head according to any one of the preceding claims, wherein the at least one image sensor device (130; 130, 650) is further configured for photographic imaging of the one or more objects (O) in the measuring field (M), wherein, as an option, the measuring head (600) further comprises an illumination device (660), in particular a white light source, configured to illuminate the one or more objects (O) in the measuring field (M) when the measuring head (600) is operated in a photographic imaging mode.

9. Measuring head according to any one of the preceding claims, wherein the at least one image sensor device (130, 650) comprises a first image sensor device (130) and at least a second image sensor device (650) arranged spatially offset from the first image sensor device (130) with respect to the measuring field (M), wherein image sensor signals (SL1, SL3) of the first image sensor device (130) and the second image sensor device (650) are further configured to be indicative of the depth profile of the one or more objects (O) in the measuring field (M) in accordance with a stereography based at least partially on the spatial offset between the first image sensor device (130) and the second image sensor device (650).

10. Measuring head according to any one of the preceding claims, further comprising a photo diode (640), in particular an avalanche photo diode, the photo diode (640) configured to receive reflected light (L_R) of the laser beam (L_D), the reflected light (L_R) reflected towards the measuring head (600) by the one or more objects (O) in the measuring field (M), wherein the photo diode (640) is configured to provide at least one photo diode signal (SL2) indicative of at least a time at which the reflected light (L_R) of the laser beam (L_D) has been received by means of the photo diode (640), wherein the at least one position signal (SP) and the at least one photo diode signal (SL2) are configured to be indicative of the depth profile of the one or more objects (O) in the measuring field (M) in accordance with a time of flight.

11. Measuring head according to any of the preceding claims, wherein the at least one laser device (110) is configured to output a pulsed laser beam (L_O, L_D).

12. Endoscope (300; 420) including a measuring head (330; 410) according to any one of the preceding claims.

13. Measuring system (400) comprising:
- a measuring head (100; 600) according to any one of claims 1 to 11 and/or an endoscope (300; 420) according to claim 12 comprising such measuring head (330; 410), and
- a data processor (442) operatively connected to the measuring head (100; 330; 410; 600), the data processor (442) configured to:
∘ receive, from the measuring head (100; 330; 410; 600), at least one position signal (SP) indicative of a variable position of the at least one variable mirror arrangement (122, 124) and at least one image sensor signal (SL1; SL1, SL3) indicative of a position of received reflected light (L_R) of the laser beam (L_D) in an image plane of the image sensor device (130; 130, 650), and
∘ process the received at least one position signal (SP) and the received at least one image sensor signal (SL1; SL1, SL3) in accordance with a triangulation based at least partially on the one or more dynamic projection patterns (DP1; DP2) to determine a depth profile of one or more objects (O) in the measuring field (M).

14. Method (500) of operating a measuring head (100; 330; 410; 600), in particular for an endoscope (300; 420), the method (500) comprising:
- providing (510) a measuring head (100; 330; 410; 600) according to any one of claims 1 to 12;
- positioning (520) the measuring head (100; 330; 410; 600) such that one or more objects (O) to be measured are in the measuring field (M) of the measuring head (100; 330; 410; 600);
- receiving (530), from the measuring head (100; 330; 410; 600), at least one position signal (SP) indicative of a variable position of the at least one variable mirror arrangement (122, 124) and at least one image sensor signal (SL1; SL1, SL3) indicative of a position of received reflected light (L_R) of the laser beam (L_D) in an image plane of the image sensor device (130, 150), and
- processing (540), by means of a data processor (442), the received at least one position signal (SP) and the received at least one image sensor signal (SL1; SL1, SL3) in accordance with a triangulation based at least partially on the one or more dynamic projection patterns (DP1; DP2) to determine a depth profile of the one or more objects (O).

15. Method according to claim 14, wherein:
- the at least one image sensor signal (SL1; SL1, SL3) is further indicative of one or more photographic characteristics, in particular one or more colors, of the one or more objects (O) in the measuring field (M), wherein processing (540) the received at least one position signal (SP) and the received at least one image sensor signal (SL1; SL1, SL3) further comprises associating the one or more photographic characteristics with the depth profile of the one or more objects (O) to generate a data set comprising a three-dimensional photographic representation of at least a portion of the one or more objects (O), and/or
- processing (540) the received at least one position signal (SP) and the received at least one image sensor signal (SL1; SL1, SL3) is performed in accordance with a calibration rule for the measuring head (100).
